# FASCICULE DE BREVET EUROPEEN

(11) **EP 0 559 768 B1**
(45) Date de publication et mention de la délivrance du brevet: **25.05.1994**
(21) Numéro de dépôt: 92900977.7
(22) Date de dépôt: 22.11.1991
(51) Int. Cl.: A61K 9/16, A61K 9/50, A61K 31/71

(54) **PROCEDE DE PREPARATION DE FORMULATION DE SPIRAMYCINE ET FORMULATION ORALES DE SPIRAMYCINE**
SPIRAMYCIN ENTHALTENDE ARZNEIMITTEL UND VERFAHREN ZU DEREN HERSTELLUNG
ORAL SPIRAMYCINE FORMULATIONS AND METHOD FOR PREPARING SAME

(30) Priorité: 26.11.1990 FR 9014726
(43) Date de publication de la demande: 15.09.1993
(73) Titulaire: RHONE-POULENC RORER S.A., 92160 Antony (FR)
(72) Inventeur: CALVO, Ana Calle Rio Tajo, 108, E-28669 Madrid (ES); DEL RIO, Luis Alberto, E-28008 Madrid (ES); ESTEBAN, Manuel Burgo de las Rozas, E-28230 Las Rozas Madrid (ES); RONA, Robert, F-78100 Saint-Germain-en-Laye (FR)
(74) Mandataire: Le Pennec, Magali
(86) Numéro de dépôt international: FR9100927
(87) Numéro de publication internationale: WO9209269

(56) Documents cités:
- GB-A- 1 413 186
- WPIL/Derwent, Accession n 87-066792, (10), Derwent Publications Ltd (London, GB) & ES-A-8700269 (RHONE POULENC PHARMA.) 1 January 1987, see the abstract

## Description

La présente invention concerne un nouveau procédé de préparation de granulés de spiramycine micoencapsulée ainsi que les formulations obtenues à partir de ces granulés.

La spiramycine ainsi que ses sels et ses esters est un antibiotique à large spectre d'activité dont l'utilisation par voie orale surtout chez l'enfant est limitée pour des problèmes de goût. En effet la spiramycine, ses sels ou ses esters présente des propriétés physiques ou chimiques qui au niveau des papilles gustatives provoquent une amertume qui entraine une difficulté d'ingestion pouvant aller jusqu'au rejet du médicament par des phénomènes de vomissements.

Chez l'adulte il a été possible de circonvenir ces inconvénients par l'administration de la spiramycine sous une forme comprimée enrobée par un polymère ou une laque qui évite tout contact du principe actif avec les papilles gustatives. Chez le jeune enfant cette solution n'est pas envisageable. L'industrie pharmaceutique est depuis longtemps à la recherche d'une forme liquide administrable chez l'enfant ou chez l'adulte permettant un masquage correct de l'amertume du principe actif même en solution.

Il est connu par le brevet d'importation espagnol numéro 550171/7 de préparer des microcapsules de spiramycine enrobée par une protéine, cette dernière est de préférence l'albumine. Il est précisé dans ce texte que les microcapsules peuvent être utilisées pour la préparation de suspensions liquides. Le procédé de préparation desdites microcapsules n'est pas facile à mettre en oeuvre, il consiste à préparer un mélange de spiramycine et d'albumine, à coaguler l'albumine par la chaleur puis à laver le solide obtenu à plusieurs reprises, à le centrifuger puis à le sécher. Il est précisé dans le texte que les microcapsules ont une forme irrégulière.

La présente invention a permis de préparer des granulés prêts à l'emploi composés de spiramycine encapsulée dans l'albumine et dilués par un mélange de sucres et d'aromatisants. Le mode de préparation a été grandement facilité par rapport au procédé décrit dans le brevet espagnol préalablement cité. En effet il ne nécessite plus d'étapes intermédiaires de lavage des microcapsules pour éliminer le solvant et permet en outre d'opérer toutes les étapes dans le même appareillage jusqu'à la formulation finale.

Ce procédé de préparation de formulations prêtes à l'emploi de spiramycine consiste:
- dans une première étape à préparer une solution d'albumine dans un tampon phosphate à pH 7,5 à 8,5 en présence d'un agent antimousse;
- dans une deuxième étape à préparer une suspension de spiramycine dans l'isooctane en présence d'un agent antimousse;
- dans une troisième étape à préparer un mélange contenant des sucres et de l'eau, à le granuler et à le sécher par agitation sous vide;
- dans une quatrième étape à mélanger dans une turbosphère la solution et la suspension des étapes 1 et 2 selon un rapport pondéral albumine sur spiramycine compris entre 0,3 et 0,5, à chauffer le mélange à une température comprise entre 35 et 40°C pour l'émulsionner, puis à le chauffer à une température supérieure pour coaguler l'albumine;
- dans une cinquième étape à évaporer sous vide les solvants du mélange précédent à une température inférieure à 65°C sous une pression inférieure à 13,33 kPa (100 torrs);
- dans une sixième étape à mélanger les granulés obtenus à la troisième étape et à la cinquième étape selon un rapport pondéral compris entre cinq pour un et cinquante pour un, en ajoutant éventuellement des arômes, des édulcorants et/ou des colorants.

Selon un mode de mise en oeuvre de l'invention au cours de la première étape on prépare notamment une solution aqueuse tamponnée à pH 7,5 - 8,5 par mélange de phosphate disodique et monopotassique. L'agent antimousse qui est ajouté à cette solution peut être d'origine chimique ou physique. L'agent physique peut être le vide, l'agent chimique peut être choisi parmi les agents tensioactifs anionique, cationique ou non ionique (éthers et/ou esters d'acide gras). On préfère utiliser l'oléate de sorbitane. Selon un mode de réalisation préféré de l'invention on ajoute environ 0,3 % en poids de trioléate de sorbitane comme agent antimousse à la solution aqueuse tamponnée et enfin on ajoute l'albumine selon un rapport pondéral par rapport à la solution aqueuse tamponnée d'environ 20 %. L'agitation est maintenue pendant quelques heures jusqu'à dissolution complète de l'albumine.

Selon un meilleur mode de mise en oeuvre de l'invention, le pH est de préférence ajusté entre 7,9 et 8,1 et la température est maintenue entre 25 et 35 °C.

Selon un procédé de mise en oeuvre de la deuxième étape de l'invention on disperse dans la turbosphère la spiramycine dans un mélange d'isooctane et d'agent antimousse, de préférence contenant environ 3,5 % en poids de trioléate de sorbitane par rapport à l'isooctane, le rapport pondéral entre la spiramycine et le mélange isooctane-trioléate de sorbitane étant compris entre 50 et 100 %. On préfére selon un meilleur mode de mise en oeuvre de l'invention utiliser un rapport pondéral de 80 %.

Selon un procédé de mise en oeuvre de la troisième étape de l'invention qui peut indifféremment être une première étape ou une cinquième étape du procédé selon invention ou ne pas exister si le granulé de sucre est disponible, on prépare un granulé de sucres, par exemple un mélange de lactose et de fructose et d'eau que l'on granule et on sèche. Cette granulation peut être effectuée dans la turbosphère ou dans tout autre mélangeur sécheur de préférence avant l'encapsulation de la spiramycine de façon à ne pas devoir vider la turbosphère en cours de fabrication. Ce ganulé est de préférence tamisé pour conserver une granulométrie inférieure à 0,4 mm.

Selon un procédé de mise en oeuvre de la quatrième étape de l'invention on introduit dans la turbosphère, contenant la spiramycine en dispersion dans l'isooctane, la solution d'albumine dans le tampon. On introduit de préférence une quantité de solution d'albumine telle que le rapport pondéral de l'albumine sur la spiramycine soit compris entre 0,3 et 0,5. Cette introduction est effectuée de façon tout à fait préférentielle en augmentant la vitesse d'agitation de la turbosphère aux environs de 70 à 90 tours par minute, la température étant fixée notamment entre 35 et 45 °C. Cette agitation permet d'obtenir un émulsion stable. Afin de ne pas détruire la protéine en cours de coagulation, on diminue avantageusement l'agitation à 10 à 30 tours par minute en maintenant la température en dessous de 65°C.

Selon la cinquième étape du procédé de l'invention on évapore sous vide les solvants du mélange précédent c'est à dire l'eau et l'isooctane à une température comprise entre 50 et 65°C. L'agitation est ralentie de préférence jusqu'à environ 5 à 10 tours par minute et le vide est appliqué lentement jusqu'à une valeur inférieure à 13,33 kPa (100 torrs) et de préférence jusqu'à une valeur comprise entre 1,33 et 5,32 kPa (10 et 40 torrs), la température étant elle maintenue de préférence en dessous de 60°C. La spiramycine encapsulée obtenue est amisée de façon à ne conserver que la granulométrie inférieure à 0,3 mm.

Selon la sixième étape du procédé selon l'invention on mélange les granulés de microcapsules obtenus à l'étape précédente avec les granulés constitués de sucre, obtenus à la troisième étape ou à une autre étape du procédé, on ajoute les agents d'aromatisation, les édulcorants et les colorants. Selon un mélange préférentiel on ajoute les granulés de sucre aux granulés de spiramycine microencapsulée selon un rapport pondéral de cinq pour un à cinquante pour un.

Les formulations orales à base de spiramycine encapsulée par l'albumine utilisables et obtenues par le procédé de l'invention sont dosées de préférence entre 150 000 à 3 000 000 d'unités internationales de spiramycine et encore plus préférentiellement elles contiennent 250000 unités internationales de spiramycine par gramme de mélange. Elles sont insipides et donc d'ingestion facile autant pour l'enfant que pour l'adulte.

La présente invention sera plus complètement décrite à l'aide de l'exemple suivant qui ne doit pas être considéré comme limitatif de l'invention.

### EXEMPLE

a) Préparation du tampon
   On mélange 107,17 kg d'eau déminéralisée (ce qui correspond à un excès de 40 % du aux pertes futures), 0,938 kg de phosphate de sodium disodique anhydre et 0,053 kg de phosphate monopotassique. Après cinq minutes d'agitation on prend un échantillon pour mesurer le pH qui doit se trouver dans la fourchette 7,9 - 8,1. Si le pH est trop élevé on ajoute du phosphate de potassium, s'il est trop bas on ajoute du phosphate de sodium.
b) Addition de l'albumine
   Dans la solution précédente on ajoute 0,407 kg de trioléate de sorbitane comme antimousse et 27,04 kg d'albumine d'oeuf en évitant la formation de grumeaux. On agite pendant trois heures jusqu'à dissolution totale. A la fin de ce temps on filtre sur un filtre de 0,2 mm pour éliminer la mousse formée.
c) Encapsulation
   On utilise une turbosphère Moritz TSI - 500 Pharma que l'on chauffe par l'intermédiaire de la double enveloppe à une température de 40°C. On ajoute 44,572 kg de spiramycine puis une charge de 75 litres d'isooctane. On met en marche les pâles de la turbosphère à une vitesse de 20 tours par minute. On charge ensuite, dissout dans 6,84 litres d'isooctane, 1,937 kg de trioléate de sorbitane. On homogénéise la suspension à 40 tours par minute pendant 10 minutes.
   On augmente la vitesse d'agitation à 70 - 90 tours par minute et on y ajoute 96,863 kg de la solution d'albumine. La durée de l'addition est de 10 à 20 minutes et la température de la solution est de 35 à 42°C. Ensuite on homogénéise l'émulsion pendant 5 à 10 autres minutes. On arrête l'agitation et on observe l'apparition d'une émulsion stable d'une couleur blanc jaune.
   Pour coaguler l'albumine on augmente la température de la double enveloppe jusqu'à 80 - 85°C et on augmente de nouveau la vitesse d'agitation jusqu'à 20 à 30 tours par minute. Quand commence une première coagulation de la masse aux alentours de 50 - 53°C, on diminue l'agitation à 15 - 20 tours par minute pour éviter une trop grande extrusion de l'isooctane de la masse. Au fur et à mesure de la coagulation de l'albumine vers 56 - 59°C, on baisse de nouveau l'agitation jusqu'à 10 - 15 tours par minute pour éviter au maximum l'extrusion de l'isooctane des microcapsules. Quand tout est coagulé aux alentours de 61°C on baisse la température de la double enveloppe de façon à modifier la température du milieu la plus lentement possible.
d) séchage
   Quand la température du milieu atteint 65°C, on applique le vide et on diminue l'agitation à 5 à 10 tours par minute. On diminue le chauffage lentement en veillant à ce que la température intérieure ne dépasse pas 65°C. La vitesse d'application du vide s'instaure de la façon la lente possible. La vitesse d'évaporation la mieux contrôlée de la masse correspond à une température intérieure de 50°C. A ce moment on peut augmenter le vide sous 13,33 kPa (100 torrs) et même jusqu'à 1,33 et 5,32 kPa (10 à 40 torrs). Quand le produit est suffisamment sec on le décharge et on le tamise sur un tamis de 0,3 mm.
e) granulés de base
   On introduit dans la turbosphère après tamisage sur un tamis de 0,4 mm, 66,822 kg de lactose et 100,233 kg de fructose. On agite à 20 tours par minute pendant 30 minutes. On ajoute 2,406 litres d'eau et on malaxe. On chauffe la turbosphère à 70 - 75°C et on applique le vide. En deux à quatre heures on obtient un granulé présentant un diamètre inférieur à 0,4 mm.
f) mélange final
   On ajoute aux granulés de sucre préalablement préparés 0,134 kg de colorant, 16,706 kg de spiramycine microencapsulée (25 % de la production), 3,341 kg d'arôme de framboise en poudre, 3,341 kg d'arôme de banane et 2,673 kg d'aspartame. Après 45 à 60 minutes on obtient une homogénéité totale. La poudre obtenue est passée sur un tamis de dimension de maille inférieur à 0,4 mm et répartie dans des conditionnements appropriés.

## Revendications

1. Procédé de préparation de formulations de granulés de spiramycine caractérisé en ce que:
- dans une première étape on prépare une solution d'albumine dans un tampon phosphate à pH 7,5 à 8,5 en présence d'un agent antimousse;
- dans une deuxième étape on prépare une suspension de spiramycine dans l'isooctane en présence d'un agent antimousse;
- dans une troisième étape on prépare un mélange contenant un ou plusieurs sucre et de l'eau, on le granule et on le sèche par agitation sous vide;
- dans une quatrième étape on mélange dans une turbosphère la solution et la suspension des étapes 1 et 2 selon un rapport pondéral albumine sur spiramycine compris entre 0,3 et 0,5, on chauffe le mélange à une température comprise entre 35 et 40°C pour l'émulsionner, puis on le chauffe à une température supérieure pour coaguler l'albumine;
- dans une cinquième étape on évapore sous vide les solvants du mélange précédent à une température comprise entre 50 et 65°C sous une pression inférieure à 13,33 kPa (100 torrs);
- dans une sixième étape on mélange les granulés obtenus à la troisième étape et à la cinquième étape selon un rapport pondéral compris entre cinq pour un et cinquante pour un, en ajoutant éventuellement des arômes, des édulcorants et/ou des colorants.

2. Procédé selon la revendication 1 caractérisé en ce que l'agent antimousse est soit le vide soit un agent tensioactif.

3. Procédé selon les revendications 1 et 2 caractérisé en ce que dans la première étape on prépare une solution d'albumine contenant environ 20 % en poids d'albumine et environ 0,3 % en poids d'oléate de sorbitane.

4. Procédé selon la revendication 1 et 2 caractérisé en ce que dans la deuxième étape on prépare une suspension contenant 50 à 100 g de spiramycine pour 100 g d'isooctane et environ 0,3 % d'oléate de sorbitane.

5. Procédé selon la revendication 1 caractérisé en ce que dans la troisième étape on prépare des granulés présentant une granulométrie inférieure à 0,4 mm.

6. Procédé selon la revendication 1 caractérisé en ce que dans la quatrième étape lors de la coagulation de l'albumine l'émulsion est chauffée à une température inférieure à 65°C.

7. Procédé selon la revendication 1 caractérisé en ce que dans la cinquième étape on évapore les solvants sous une pression comprise entre 1,33 et 5,32 kPa (10 et 40 torrs).

8. Procédé selon les revendications 1 et 7 caractérisé en ce que postérieurement à la cinquième étape on effectue un tamisage pour conserver les granulés ayant une granulométrie inférieure à 0,3 mm.

9. Procédé selon la revendication 1 caractérisé en ce que dans la sixième étape on mélange les granulés obtenus à la troisième étape avec ceux obtenus à la cinquième étape selon un rapport pondéral compris entre cinq pour un et cinquante pour un.

10. Formulation orale composée de spiramycine ou d'un de ses dérivés encapsulée dans de l'albumine susceptible d'être obtenue par le procédé selon l'une quelconque des revendications 1 à 9, de sucre, d'aromatisants et d'édulcorant, dosée à 250 000 unités internationales de spiramycine par gramme de mélange.

## Patentansprüche

1. Verfahren zur Herstellung granulierter Formulierungen von Spiramycin, dadurch gekennzeichnet, daß man
- in einer ersten Stufe eine Lösung von Albumin in einem Phosphat-Puffer bei pH 7,5 bis 8,5 In Anwesenheit eines Antischaummittels herstellt;
- in einer zweiten Stufe eine Suspension von Spiramycin in Isooctan in Anwesenheit eines Antischaummittels herstellt;
- in einer dritten Stufe eine Mischung herstellt, die einen oder mehrere Zucker und Wasser enthält, und sie durch Rühren unter Vakuum granuliert und trocknet;
- in einer vierten Stufe die Lösung und die Suspension der Stufen 1 und 2 gemäß einem Gewichtsverhältnis von Albumin zu Spiramycin zwischen 0,3 und 0,5 in einer Turbosphäre mischt, die Mischung auf eine Temperatur zwischen 35 °C und 40 °C erhitzt, um sie zu emulgieren, und anschließend auf eine höhere Temperatur erhitzt, um des Albumin zu koagulieren;
- in einer fünften Stufe unter Vakuum die Lösungsmittel der vorstehenden Mischung bei einer Temperatur zwischen 50 °C und 65 °C und bei einem Druck von unter 13,33 kPa (100 Torr) verdampft;
- in einer sechsten Stufe die in der dritten und in der fünften Stufe erhaltenen Granulate gemäß einem Gewischtsverhältnis zwischen 5:1 und 50:1 mischt, wobei man gegebenenfalls Aromastoffe, Süßstoffe und/oder Farbstoffe zusetzt.

2. Verfahren nach Anspruch 1, dadurch gekennzeichnet, daß das Antischaummittel entweder Vakuum oder ein oberflächenaktives Mittel ist.

3. Verfahren nach Anspruch 1 und 2, dadurch gekennzeichnet, daß man in der ersten Stufe eine Albumin-Lösung herstellt, die etwa 20 Gew.-% Albumin und etwa 0.3 Gew.-% Sorbitanoleat enthält.

4. Verfahren nach Anspruch 1 und 2, dadurch gekennzeichnet, daß man in der zweiten Stufe eine Suspension herstellt, die 50 g bis 100 g Spiramycin pro 100 g Isooctan und etwa 0.3 Gew.-% Sorbitanoleat enthält.

5. Verfahren nach Anspruch 1, dadurch gekennzeichnet, daß man in der dritten Stufe Granulate herstellt, die eine Granulometrie von unterhalb 0,4 mm aufweisen.

6. Verfahren nach Anspruch 1, dadurch gekennzeichnet, daß in der vierten Stufe bei der Koagulation des Albumins die Emulsion auf eine Temperatur von unterhalb 65 °C erhitzt wird.

7. Verfahren nach Anspruch 1, dadurch gekennzeichnet, daß man in der fünften Stufe die Lösungsmittel unter einem Druck zwischen 1,33 und 5,32 kPa (10 und 40 Torr) verdampft.

8. Verfahren nach Anspruch 1 und 7, dadurch gekennzeichnet, daß man nach der fünften Stufe eine Siebung durchführt, um die Granulate mit einer Granulometrie von unter 0,3 mm zurückzuhalten.

9. Verfahren nach Anspruch 1, dadurch gekennzeichnet, daß man in der sechsten Stufe die in der dritten Stufe erhaltenen Granulate mit den in der fünften Stufe erhaltenen Granulaten gemäß einem Gewichtsverhältnis zwischen 5:1 und 50:1 mischt.

10. Orale Formulierung, zusammengesetzt aus Spiramycin oder einem seiner Derivate, eingekapselt in Albumin, die nach dem Verfahren gemäß irgendeinem der Ansprüche 1 bis 9, mit Zucker, Aromastoffen und Süßstoff in einer Dosierung von 250.000 Internationalen Einheiten Spiramycin pro Gramm Mischung erhalten werden kann.

## Claims

1. Process for the preparation of formulations of spiramycin granules, characterized in that:
- in a first stage, an albumin solution is prepared in a phosphate buffer at pH 7.5 to 8.5 in the presence of an antifoaming agent;
- in a second stage, a spiramycin suspension is prepared in isooctane in the presence of an antifoaming agent;
- in a third stage, a mixture is prepared containing one or more sugars and water, it is granulated and it is dried by stirring under vacuum;
- in a fourth stage, the solution and the suspension of stages 1 and 2 are mixed in a turbosphere in an albumin to spiramycin weight ratio of between 0.3 and 0.5, the mixture is heated to a temperature of between 35 and 40°C in order to emulsify it, and then it is heated to a higher temperature in order to coagulate the albumin;
- in a fifth stage, the solvents of the preceding mixture are evaporated under vacuum at a temperature of between 50 and 65°C, under a pressure of less than 13.33 kPa (100 torr);
- in a sixth stage, the granules obtained in the third stage and in the fifth stage are mixed in a weight ratio of between five to one and fifty to one, flavourings, sweeteners and/or colorants being optionally added.

2. Process according to Claim 1, characterized in that the antifoaming agent is either the vacuum or a surfactant.

3. Process according to Claims 1 and 2, characterized in that in the first stage, an albumin solution is prepared containing about 20 % by weight of albumin and about 0.3 % by weight of sorbitan oleate.

4. Process according to Claims 1 and 2, characterized in that in the second stage, a suspension is prepared containing 50 to 100 g of spiramycin per 100 g of isooctane and about 0.3% sorbitan oleate.

5. Process according to Claim 1, characterized in that in the third stage, granules are prepared having a particle size of less than 0.4 mm.

6. Process according to Claim 1, characterized in that in the fourth stage, during the coagulation of the albumin, the emulsion is heated to a temperature of less than 65°C.

7. Process according to Claim 1, characterized in that in the fifth stage, the solvents are evaporated under a pressure of between 1.33 and 5.32 kPa (10 and 40 torr).

8. Process according to Claims 1 and 7, characterized in that subsequent to the fifth stage, sifting is performed in order to retain the granules having a particle size of less than 0.3 mm.

9. Process according to Claim 1, characterized in that in the sixth stage, the granules obtained in the third stage are mixed with those obtained in the fifth stage in a weight ratio of between five to one and fifty to one.

10. Oral formulation composed of spiramycin or one of its derivatives, encapsulated in albumin capable of being obtained by the process according to any one of Claims 1 to 9, sugar, flavouring agents and sweetener, containing a dose of 250,000 international units of spiramycin per gram of mixture.
